# EUROPEAN PATENT SPECIFICATION

(11) **EP 2 205 948 B1**
(45) Date of publication and mention of the grant of the patent: **27.07.2016**
(21) Application number: 08845599.3
(22) Date of filing: 24.10.2008
(51) Int. Cl.: G01G 23/37, A01K 5/01, G06F 19/00, G01G 19/414, A01K 5/00, A01K 29/00

(54) **REMOTE DATA COLLECTING SYSTEM AND METHOD**
FERNDATENSAMMELSYSTEM UND VERFAHREN
SYSTÈME ET PROCÉDÉ DE COLLECTE DE DONNÉES À DISTANCE

(30) Priority: 01.11.2007 US 1330
(43) Date of publication of application: 14.07.2010
(73) Proprietor: Nestec S.A., 1800 Vevey (CH)
(72) Inventor: CHANG, David, Beijing 101302 (CN); MA, Changpu, Shanghai 200442 (CN)
(74) Representative: Rupp, Christian
(86) International application number: PCT/EP2008/009027
(87) International publication number: WO 2009/056260

(56) References cited:
- EP-A1- 0 819 921
- EP-A1- 1 239 268
- FR-A1- 2 839 148
- US-A1- 2001 034 671
- US-A1- 2009 187 349
- "WAEGE-ELEKTRONIK GEHT INS NETZ" WDM : WAGEN DOSIEREN + MISCHEN, VERLAG COATING THOMAS & CO., ST. GALLEN, CH, vol. 32, no. 1, 1 February 2001 (2001-02-01), page 25, XP000992086 ISSN: 1435-2176

## Description

### CROSS REFERENCE TO RELATED APPLICATIONS

This application claims priority to U.S. Provisional Application Serial No. 61/001,330 filed November 01, 2007.

### BACKGROUND OF THE INVENTION

### Field of the Invention

The invention relates generally to remote data collecting systems and methods and particularly to in-home remote data collecting systems and methods.

### Description of Related Art

Product testing generally takes place in a controlled environment such as a laboratory or research facility. Products such as human or pet foods can be conveniently tested at these locations because of the instruments and tools available to determine related parameters such as product nutritional quality, product physical characteristics, animal consumption rates, and consumption times. Similarly, the palatability of human or pet foods is generally tested in controlled environments. However, testing products such as foods for palatability or other parameters in a controlled environment such as a laboratory or research facility has limitations. The tests may not accurately reproduce the actual environment where the foods will be consumed. For example, a test subject's actual surroundings may affect their perceptions and taste preferences. Similarly, controlled environments such as a laboratory may not be available for needed testing. Circumstances may require that such tests be conducted in multiple locations that are inconvenient for those conducting the tests, e.g., in-home palatability tests for pet foods. Therefore, there is a need for remote data collecting systems that collect data from uncontrolled environments, including systems for collecting data from one or more in-home environments or environments that are situated at a long distance. US 2001/0034671 A1 discloses a method and device for monitoring inventory. EP1 239 268 A1 discloses an Internet based weighing system.

### SUMMARY OF THE INVENTION

The present invention is generally directed to remote data collecting systems and methods. In one embodiment, the present invention provides a remote data collecting system comprising a measuring device comprising at least two scales in communication with a terminal center and a control center in communication with the terminal center. Each of the scales comprises a receptacle for holding a product. The control center has the ability to collect and distribute data related to product removed from the receptacle of each scale. In various embodiments, the product is a food, preferably a pet food. The system is particularly useful for testing the palatability of foods, including pet foods.

In one embodiment, each of the scales comprises a component such as a weight sensor, a battery power pack, a liquid crystal display, a battery, a circuit board, or combinations thereof. In another, the scales are removably attached to each other.

In an embodiment, the terminal center comprises a circuit board and two communication modules for collecting, screening and distributing data between the scales and the control center.

In one embodiment, the control center comprises a circuit board and a communication module for collecting and distributing data. The control center can be linked to a computer with software for organizing, analyzing and displaying data.

In another embodiment, the present invention provides a method of collecting data remotely. This method comprises providing a measuring device comprising at least two scales in communication with a terminal center and a control center in communication with the terminal center. Each of the scales contains a product. Data is collected with respect to the amount of one or more products removed from each of the scales.

In an embodiment, the data is collected by preset sampling time and/or weight changes for a predetermined amount of time. In another, the data is transferred from the measuring device to the terminal center through a low frequency wireless connection.

In one embodiment, the method comprises displaying the data collected in the form of a graph.

In one embodiment, the control center collects and distributes the data. In another, the control center is capable of conducting a plurality of trials for determining amounts of product removed from each of the scales.

In an embodiment, the measuring device shuts off automatically when not in use.

In an alternative embodiment, the present invention provides a method of testing the palatability of pet food remotely. This method comprises providing a measuring device comprising at least two scales in communication with a terminal center and a control center in communication with the terminal center. Each of the scales comprises a different pet food. A pet is introduced to the pet foods on the measuring device. Data is collected with respect to the amount of pet food eaten by the pet from each of the scales.

In an embodiment, the method comprises collecting data by preset sampling time and/or weight changes for a predetermined amount of time. In another, the method comprises collecting data in preset conditions for a predetermined amount of time. In a further, the method comprises displaying the data collected in the form of a graph.

In another embodiment, the present invention provides a method of testing the palatability of pet food in a domestic or in-home environment. This method comprises providing a measuring device comprising at least two scales in communication with a terminal center and a control center in communication with the terminal center. The measuring device is located at the domestic environment, typically in the home of a pet caregiver. Each of the scales comprises a different pet food. A pet is introduced to the pet foods on the measuring device at the domestic location. Data is collected with respect to the amount of pet food eaten by the pet from each of the scales.

In an embodiment, the domestic environment is selected from the group consisting of a house, a store, a vehicle, and combinations thereof.

An advantage of the present invention is to provide improved methods for testing products. Another is to provide improved remote data collecting systems and methods. Yet another advantage is to provide an improved system for testing food palatability. A further advantage is to provide an improved system and method for testing food palatability remotely.

Additional features and advantages of the invention will be readily apparent to those skilled in the art.

### BRIEF DESCRIPTION OF THE DRAWINGS

FIG. 1 illustrates the remote data collecting system in an embodiment of the invention.
FIG. 2 illustrates two removably attached scales of the remote data collecting system in an embodiment of the invention.
FIG. 3 illustrates a top perspective view of a scale of the remote data collecting system in an embodiment of the invention.
FIG. 4 illustrates a bottom perspective view of a scale of the remote data collecting system in an embodiment of the invention.
FIG. 5 illustrates a bowl and the internal components of a scale of the remote data collecting system in an embodiment of the invention.
FIG. 6 illustrates the internal components of a scale of the remote data collecting system in an embodiment of the invention.

### DETAILED DESCRIPTION OF THE INVENTION

The present invention is generally directed to remote data collecting systems. For example, the remote data collecting system can provide information with respect to an amount of product (e.g., human or pet food) consumed/removed at a specified location and comparing the preferences of the consumer for the products. This can be accomplished by recording the weight differences of the product with time. In an embodiment, the remote monitoring of pet or other animals (e.g., dog/cat) eating behavior and food consumption can be performed.

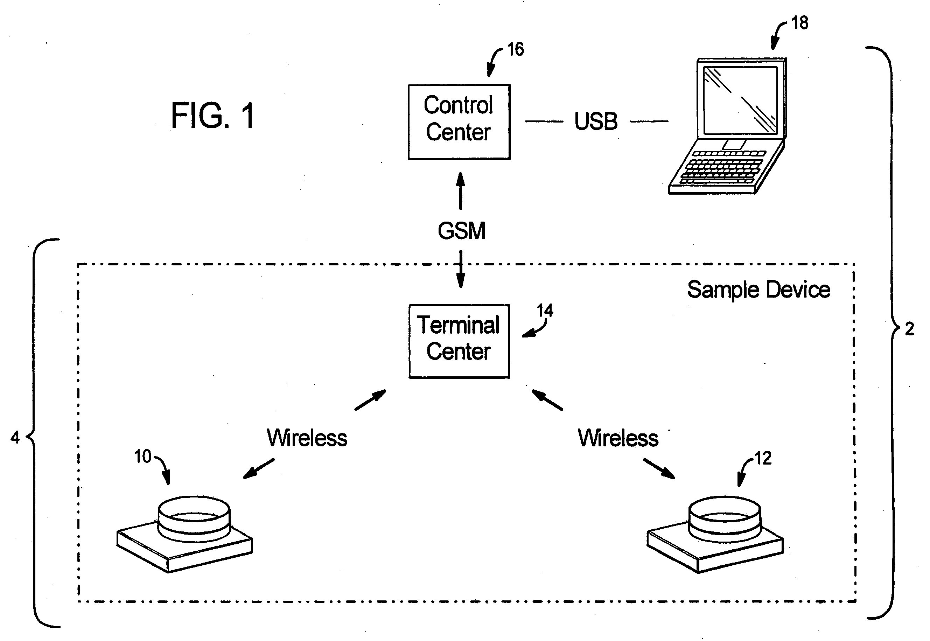

In a general embodiment illustrated in FIG. 1, the present invention provides a remote data collecting system 2 comprising a measuring device 4 comprising a first scale 10 and a second scale 12 and a terminal center 14. Each of the scales 10 and 12 is in communication with a terminal center 14. The terminal center 14 is in communication with a control center 16. The control center 16 can collect and distribute data from the measuring device 4. The scales 10 and 12 are in communication with the terminal center 14 via any suitable wired or wireless connection. Similarly, the control center 16 is in communication with the terminal center 14 via any suitable wired or wireless connection.

The control center 16 can be linked to a computer 18 with appropriate software for organizing, analyzing and displaying the data. In another embodiment, the control center and the computer for organizing and displaying the data can comprise a single unitary device.

In an embodiment, the remote data collecting system 2 can measure food intake data and transmit such data to the control center 16 for analysis, for example, using wireless transmitting means such as Global System for Mobile communications (GSM), General Packet Radio Service (GPRS) or any mobile phone system. The scales 10 and 12 can measure food consumption by comparing the amount (*e*.*g*., by weight) of food on the scales 10 and 12 before and after a meal. The scales 10 and 12 send the food consumption data to the terminal center 14, for example, through a low frequency wireless connection. The terminal center 14 sends the data to the control center 16 after collection. The data from the control center 16 can then be analyzed to determine palatability or similar information, for example, through a computing means such as the computer 18 programmed to analyze the data.

In another embodiment, the control center or the computer can be used to initiate testing periods and record testing data in real time or periodically in desirable intervals. Thus, not only is the consumption of food measured, the rate of consumption can be measured. The remote data collecting system can be designed to avoid false positives, for example, if one or more of the scales are stepped upon.

In an alternative embodiment, a mobile phone or other wired or wireless system (*e*.*g*., internet) can be used as a control center. For example, the mobile phone can be sold as part of the remote data collecting system to pet owners who can monitor pet food consumption from anywhere if the pet owners are concerned about a pet at home. The mobile phone or other wired or wireless system can be set up so that the pet owners can send a command or request to the remote data collecting system, for example, using the short message service (SMS) capabilities of the phone. In addition, when the terminal center detect one of the scales is not working, the terminal center can send a short message to the mobile phone to remind the pet owner to check the scale or to turn the scale on. In another embodiment, it is possible to combine a radio, video and picture capture system with the remote data collecting system so that the pet owner can communicate with their pet.

The measuring device 4 can record the amount of product removed or consumed from the scales 10 and 12 and store the data into a memory card inside the scales 10 and 12. The data can then be transferred through a GPRS/GSM and/or Code Division Multiple Access CDMA (*e*.*g*., any mobile phone system) or internet network to the control center 16 automatically at any specified time period and/or after a trial is completed. These devices can use a Subscriber Identity Module (SIM) card that could allow communication over a long distance *(e.g.,* worldwide communication). A short distance system like radio communication can also be used.

As previously discussed, in an embodiment, the measure device 4 comprises the terminal center 14 and two scales: the first scale 10 and the second scale 12. It should be appreciated that the measuring device can comprise more than two scales and operate in a similar manner. Any amount of product removed from each of the plurality of scales can be determined and compared. In an alternative embodiment, the terminal center can be a part of one or more of the scales (*e.g.,* as a singular device).

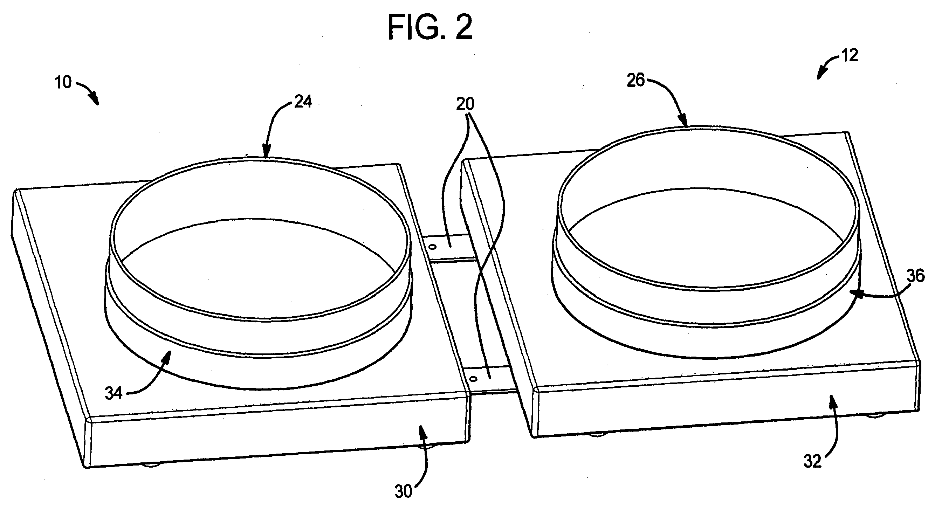

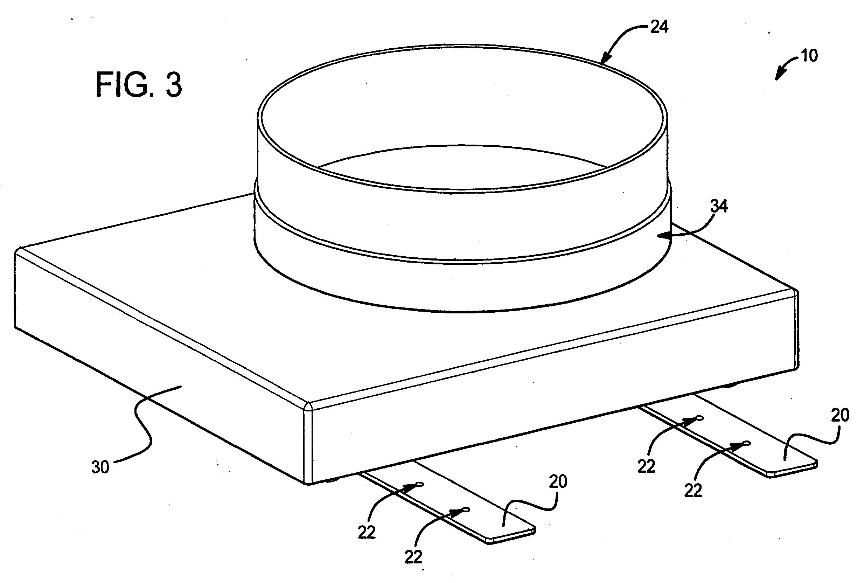

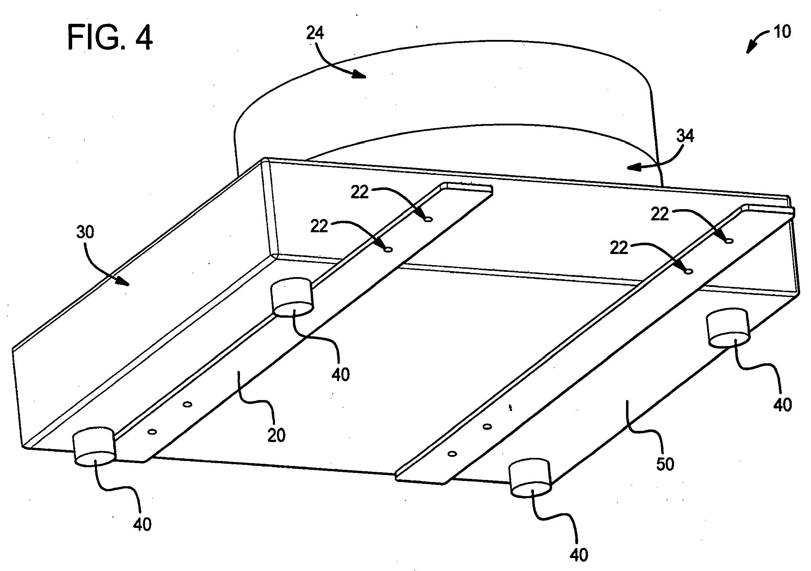

In a general embodiment illustrated in FIGS. 2-4, the scales 10 and 12 can be removably connected together through one or more connecting sticks 20, which can provide any specified distance between the scales 10 and 12. The sticks 20 can define one or more holes 22 to easily allow the distance between the scales 10 and 12 to be manually or automatically adjusted.

The scales 10 and 12 can further comprise one or more bowls 24 and 26 placed on top of the scales 10 and 12. The bowls 24 and 26 can be removably or permanently attached to the scales 10 and 12, respectively. In an embodiment, the bowls can be placed and fixed to the scales through a magnet system, which prevents the bowls from being moved from the scales by the consumer or pet.

Each of the scales 10 and 12 can comprise a scale top cover 30 and 32 made of any suitable material such as, for example, stainless steel. The material can be specified for any hygienic requirements. Each of the covers 30 and 32 can comprise a bowl holder 34 and 36, which can be used to position the bowls 24 and 26 directly over weight sensors in the scales 10 and 12 underneath to bowls 24 and 26. The scales 10 and 12 can further comprise feet 40 to keep the scales 10 and 12 stationary on any type of floor.

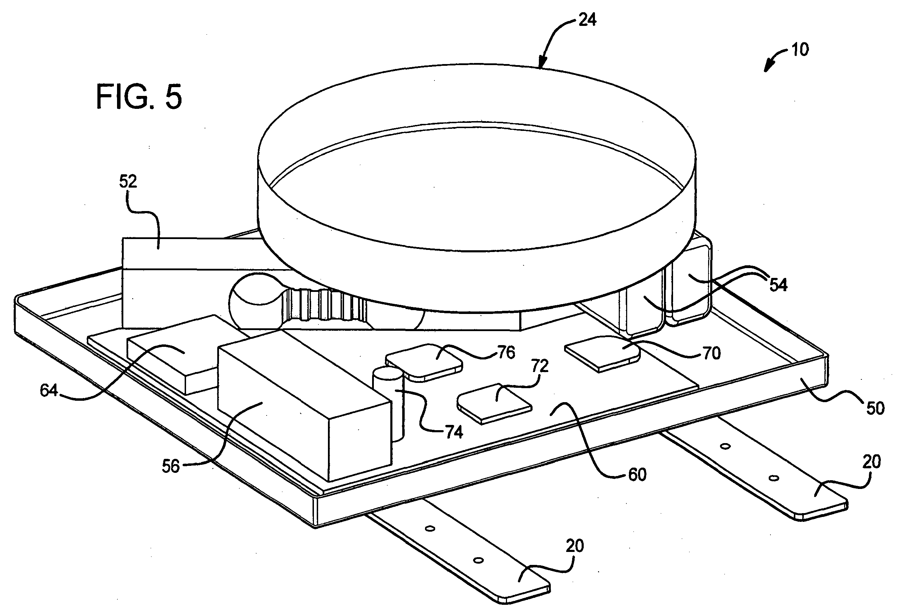

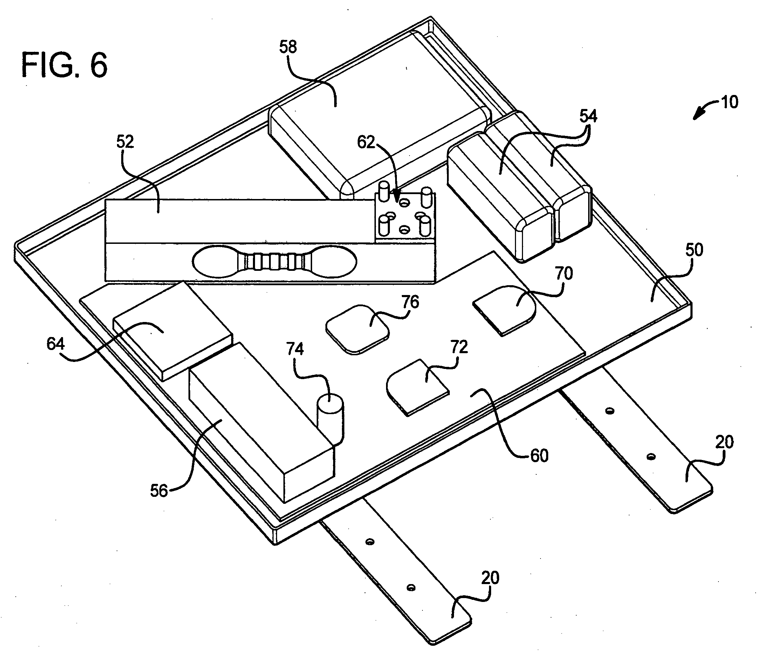

In an embodiment illustrated in FIGS. 5-6, the scale 10 (along with 12) comprises a base 50, a weight sensor 52, a battery power pack 54, a liquid crystal display (LCD) 56, a battery 58 (*e*.*g*., regular or rechargeable) and a circuit board 60. The weight sensor 52 can comprise a bowl holder stick 62 fixed on the end of the sensor 52. The circuit board 60 can comprise an analog to digital (A/D) converter module 70, a micro control unit (MCU) module 72, a light emitting diode (LED) 74 and embedded memory 76.

It should be appreciated that the scales do not need to comprise every single one of these components. In alternative embodiments, the scales can comprise any one or more of the previously described components according to the objectives of the remote data collecting system.

During operation in an embodiment, for example, when the remote data collecting system 2 is initiated, weight sensor 52 sends a signal out through the A/D converter module 70 and then to the MCU module 72 on the circuit board 60. After a calculation, weight data is sent to the terminal center 14 via a suitable wired or a wireless module 64. The terminal center 14 then sends the weight data to the control center 16 as programmed or requested.

In another embodiment, weight data can be shown on the LCD 56 and/or kept in the embedded memory 76. The LED 74 can display the scales' 10 and 12 working status and alert the user when something unexpected happens. The battery 58 provides power to the scales 10 and 12 and a battery power pack 54 can be used in case the batteries 58 run out of power. It should be appreciated that the scales can also be designed to be connected to a wall outlet or other external power source in addition to or in place of the battery power pack 54 and battery 58. In an embodiment, the main power or external supply can be used as first priority then followed by a rechargeable battery and/or regular battery.

In another aspect, the present invention provides a method of collecting data remotely. This method comprises providing a measuring device comprising at least two scales in communication with a terminal center. A control center is in communication with the terminal center. Each of the scales comprises one or more products. Data can be collected about the amount of product removed from each of the scales and sent to the control center. If the remote communication is not functioning properly, the removable memory card can be used to replace the wireless data collection function.

In another aspect, the present invention provides a method of testing the palatability of pet food in a domestic environment. As used herein, the term "domestic environment" means a location such as a consumer's house, a store, a vehicle, etc., where product research or testing is not typically performed. This method comprises providing a measuring device comprising at least two scales in communication with a terminal center and a control center in communication with the terminal center. The measuring device is located at the domestic environment. Each of the scales comprises a different pet food. A pet is introduced to the pet foods on the measuring device at the domestic location. Data is collected with respect to the amount of pet food eaten by the pet from each of the scales at the domestic environment.

Any suitable data can be collected and/or recorded from the measuring device. The data can comprise date of trial and amount of food eaten from each scale (*e.g.,* amount eaten, beginning and ending eating time). The data analysis can include the total food consumption for each specific pet and the average amount eaten over time.

The control center can collect and distribute the data to a computer. The computer and software system can analyze and display the data any suitable analysis methods or procedures. The data can be outputted in a spreadsheet or note pad format. The data can be displayed in the form of a graph, for example, comparing the amount of product removed from each of the scales over a predetermined amount of time.

In an embodiment, to graphically display trial results of pet food palatability tests, home feeding results for various animals are transmitted through a wireless network or via a Universal Serial Bus (USB) or other suitable connection to a computer system containing software that can convert the data into a graphical format. This is illustrated in FIG 1 as a connection between 16 and 18. The results are displayed on a system data analysis display screen. The display screen shows product consumption profiles and the product that was selected first by each animal when given a choice between two products.

The system operator can select a specific animal and view the display for that animal. For example, if a group of animals (*e*.*g*., 40 dogs or 30 cats) completed the same feeding trial, the group one day data can be analyzed statistically by the system software. A data analysis screen shows the left and right bowls average weight consumption. A graphic bar line on the display shows percentage of left bowl average consumptions versus percentage of right bowl average consumption. The statistical data results for the product first consumed by the animal and product preference by group is shown by *p*-values. The p-value provides information if the tested product is significantly different from the reference product.

The data can be collected over any suitable predetermined amount of time and during any number of discrete intervals within the time period. For example, the data can be collected every second or every minute for a predetermined amount of time.

The control center is capable of conducting a plurality of trials for determining amounts of product removed from each of the scales. For example, the control center can send a request for data to the measuring device at any time, and the measuring device will send back the data upon request. The data can be classified or defined by the control center. Each trial can be programmed through the control center. The duration of the trial can be adjustable from 10 min to 24 hours or any suitable amount of time.

In alternative embodiments, if the measuring device is not working properly or an unknown problem occurs, a red light can be shown automatically on the scales. A buzzer alarm can remind the pet owner to inform the control center and/or the LCD screen shows "Out of Order - Check With Your Contact Person." If the measuring device encounters minor problems that can be solved by pet owner (*e*.*g*., low battery), a yellow light can be shown on the scales. A buzzer alarm can generate a signal to inform the pet owner. Troubleshooting procedures can be shown on LCD screen. A message regarding problems on the specific device can be sent to control center in the mean time. The measuring device can also be made to shut off automatically when not in use.

In another aspect, the invention provides a remote data collecting system comprising a measuring device comprising at least two scales in communication with a terminal center, the scale comprising a receptacle for holding a product; and a control center in communication with the terminal center, the control center having the ability to collect and distribute data related to product removed from the receptacle of the scale. The system is useful for remotely collecting data related to the removal of product from the scale, particularly measuring the removal of product over time and removal frequency.

In another aspect, the present invention provides a means for communicating information about or instructions for one or more of (1) using a remote data collecting system comprising a measuring device comprising at least two scales in communication with a terminal center, the scales comprising a receptacle for holding a product; and a control center in communication with the terminal center, the control center having the ability to collect and distribute data related to product removed from the receptacle of each scale to remotely collect data relating to a product, (2) using the remote data collecting system to determine consumer preferences for a food product, and (3) using the remote data collecting system to determine palatability of a human or non-human food composition, preferably a pet food, most preferably a dog or cat pet food. The means comprises a document, digital storage media, optical storage media, audio presentation, or visual display containing the information or instructions. In certain embodiments, the communication means is a displayed web site, visual display, brochure, product label, package insert, advertisement, handout, public announcement, audiotape, videotape, DVD, CD-ROM, computer readable chip, computer readable card, computer readable disk, computer memory, or combination thereof containing such information or instructions. Useful information includes one or more of (1) methods and techniques for setting-up, using, and monitoring the system, particularly methods for placing product on the system scales, and (2) contact information for animals or their caregivers to use if they have a question about the system and its use. Useful instructions include methods for loading food onto the scales of the system. The communication means is useful for instructing on the benefits of using the system.

### EXAMPLES

The invention can be further illustrated by the following example, although it will be understood that this example is included merely for purposes of illustration and is not intended to limit the scope of the invention unless otherwise specifically indicated.

### Example 1

Data is collected using the remote data collecting system according to the following protocol:
- Connect a first scale to a second scale (the remote data collecting system can also work with only one scale or more than two scales).
- Switch on the scales. The system will perform calibration automatically. The software records one signal - "Trial start" - A green light on the scales turns on.
- Place designated feeding bowl with food on the scales. The starting weight with time will be recorded by the embedded software memory card.
- Detect weight changes every second or for a specific time period. The detected weight will be compared with the previous record. If the data detected is more than the previous data and within a certain range, no data will be recorded into the memory card. If the data detected is less than the previous weight, or if the weight is out of the certain range, it will be recorded into the memory card.
- Perform shut down function through the control center. Once the trial stops, all data will be remotely delivered to the control center, and the device will be shut down automatically. The data can be used to select products to market, to modify products based on the testing, to compare products for comparative advertising or any other suitable purpose.

In the specification, there have been disclosed typical preferred embodiments of the invention and, although specific terms are employed, they are used in a generic and descriptive sense only and not for purposes of limitation, the scope of the invention being set forth in the claims. Obviously many modifications and variations of the invention are possible in light of the above teachings. It is therefore to be understood that within the scope of the appended claims the invention may be practiced otherwise than as specifically described.

## Claims

1. A remote data collecting system, comprising:
a measuring device (4) comprising at least two scales (10, 12) in communication with a terminal center (14), the scales (10, 12) comprising a receptacle for holding a product;
a control center (16) in communication with the terminal center (14), the control center (16) having the ability to collect and distribute data related to product removed from the receptacle of each scale and
a computer (18) with a software for organizing, analyzing, and displaying the data, wherein
each of the at least two scales (10, 12) communicates with the control center (16) only via the terminal center (14) and the computer (18) with the software for organizing, analyzing, and displaying the data communicates with the terminal center (14) only via the control center (16).

2. The system of claim 1, wherein
a) each of the receptacles comprises a bowl for holding a product; optionally wherein the product comprises a pet food; or
b) each of the scales (10, 12) comprises a component selected from the group consisting of a weight sensor, a battery power back, a liquid crystal display, a rechargeable or regular battery, a circuit board, and combination thereof; or
c) the scales (10, 12) are removably attached to each other.

3. A method of collecting data remotely comprising:
providing a measuring device (4) comprising at least two scales (10, 12) in communication with a terminal center (14), a control center (16) and a computer (18) with a software for organizing, analyzing, and displaying the data,
wherein each of the at least two scales (10, 12) communicates with the control center (16) only via the terminal center (14) and the computer (18) with the software for organizing, analyzing, and displaying the data communicates with the terminal center (14) only via the control center (16), wherein each of the scales (10, 12) comprises a product; and
collecting data with respect to the amount of product removed from each of the scales (10, 12).

4. The method of claim 3, wherein
a) the control center (16) collects, distributes, and analyzes the data; or
b) the method comprises displaying the data collected in the form of a graph; or
c) the data is collected for a predetermined amount of time; or
d) the measuring device (4) shuts off automatically after a predetermined time period when not in use; or
e) the control center (16) is capable of conducting a plurality of trials for determining amounts of product removed from each of the scales; or
f) the data is transferred from the measuring device (4) to the terminal center (14) through a low frequency wireless connection; or
g) each of the scales (10, 12) comprises a bowl for holding the product; or
h) each of the scales (10, 12) comprises a component selected from the group consisting of a weight sensor, a battery power pack, a liquid crystal display, a battery, a circuit board, and combinations thereof; or
i) the scales (10, 12) are removably attached to each other; or
j) the product comprises a pet food.

5. The method according to claim 3, wherein each of the scales comprises as the product a different pet food, the method further comprising the step of introducing a pet to the pet foods on the scales(10, 12),
wherein the collecting data with respect to the amount of product removed from each of the scales (10, 12) is
collecting data with respect to the amount of pet food eaten by the pet from each of the scales (10, 12).

6. The method of claim 5, wherein the control center (16) collects and distributes the data.

7. The method of claim 5, comprising displaying the data collected in the form of a graph.

8. The method of claim 5, comprising collecting data every second for a predetermined amount of time.

9. The method of claim 5, comprising collecting data every minute for a predetermined amount of time.

## Patentansprüche

1. System zur Ferndatenerfassung, umfassend:
eine Messvorrichtung (4) umfassend mindestens zwei mit einer zentralen Datenstation (14) in Verbindung stehenden Waagen (10, 12), wobei die Waagen (10, 12) einen Behälter zur Aufnahme eines Produkts umfassen;
eine mit der zentralen Datenstation (14) in Verbindung stehende Steuerzentrale (16), wobei die Steuerzentrale (16) Daten erfassen und verteilen kann, die mit dem aus dem Behälter der einzelnen Waagen entnommenen Produkt in Zusammenhang stehen, sowie einen Computer (18) mit einer Software zum Organisieren, Analysieren und Anzeigen der Daten, wobei
jede der mindestens zwei Waagen (10, 12) mit der Steuerzentrale (16) nur über die zentrale Datenstation (14) kommuniziert, und der Computer (18) mit der Software zum Organisieren, Analysieren und Anzeigen der Daten mit der zentralen Datenstation (14) nur über die Steuerzentrale (16) kommuniziert.

2. System nach Anspruch 1, wobei
a) jeder der Behälter eine Schale zur Aufnahme eines Produkts umfasst; wobei das Produkt optional Tiernahrung umfasst; oder
b) jede der Waagen (10, 12) eine Komponente, ausgewählt aus der Gruppe bestehend aus einem Gewichtssensor, einem Akku, einer LCD-Anzeige, einer wiederaufladbaren oder normalen Batterie, einer Leiterplatte und Kombinationen von diesen umfasst; oder
c) die Waagen (10, 12) trennbar miteinander verbunden sind.

3. Verfahren zur Ferndatenerfassung, umfassend:
Bereitstellen eines Messgeräts (4), das mindestens zwei mit einer zentralen Datenstation (14) in Verbindung stehende Waagen (10, 12), eine Steuerzentrale (16) und einen Computer (18) mit einer Software zum Organisieren, Analysieren und Anzeigen der Daten umfasst,
wobei jede der mindestens zwei Waagen (10, 12) mit der Steuerzentrale (16) nur über die zentrale Datenstation (14) kommuniziert, und der Computer (18) mit der Software zum Organisieren, Analysieren und Anzeigen der Daten mit der zentralen Datenstation (14) nur über die Steuerzentrale (16) kommuniziert, wobei jede der Waagen (10, 12) ein Produkt umfasst, und
Daten in Bezug auf die Produktmenge erfasst, die jeder der Waagen (10, 12) entnommen wurde.

4. Verfahren nach Anspruch 3, wobei
a) die Steuerzentrale (16) Daten erfasst, verteilt und analysiert; oder
b) das Verfahren das Anzeigen der erfassten Daten in Form einer Grafik umfasst; oder
c) die Daten für einen vorgegebenen Zeitraum erfasst werden; oder
d) die Messvorrichtung (4) nach einem vorgegebenen Zeitraum, in dem sie nicht genutzt wurde, automatisch abschaltet; oder
e) die Steuerzentrale (16) eine Vielzahl von Tests zur Bestimmung der jeder der Waagen entnommenen Produktmengen durchführen kann; oder
f) die Daten von der Messvorrichtung (4) zur zentralen Datenstation (14) durch eine niederfrequente drahtlose Verbindung übertragen werden; oder
g) jede der Waagen (10, 12) eine Schale zur Aufnahme des Produkts umfasst; oder
h) jede der Waagen (10, 12) eine Komponente, ausgewählt aus der Gruppe bestehend aus einem Gewichtssensor, einem Akku, einer LCD-Anzeige, einer Batterie, einer Leiterplatte und Kombinationen von diesen umfasst; oder
i) die Waagen (10, 12) trennbar miteinander verbunden sind; oder
j) das Produkt Tiernahrung umfasst.

5. Verfahren nach Anspruch 3, wobei jede der Waagen als Produkt eine andere Tiernahrung umfasst, wobei das Verfahren ferner den Schritt des Heranführens eines Haustiers an die Tiernahrungsprodukte auf den Waagen (10, 12) umfasst,
wobei das Erfassen von Daten in Bezug auf die jeder der Waagen (10, 12) entnommene Produktmenge
ein Erfassen von Daten in Bezug auf die Menge Tiernahrung darstellt, die von dem Haustier aus jeder der Waagen (10, 12) gefressen wurde.

6. Verfahren nach Anspruch 5, wobei die Steuerzentrale (16) die Daten erfasst und verteilt.

7. Verfahren nach Anspruch 5, welches das Anzeigen der erfassten Daten in Form einer Grafik umfasst.

8. Verfahren nach Anspruch 5, welches das sekündliche Erfassen von Daten über einen vorgegebenen Zeitraum umfasst.

9. Verfahren nach Anspruch 5, welches das minütliche Erfassen von Daten über einen vorgegebenen Zeitraum umfasst.

## Revendications

1. Système de collecte de données à distance, comprenant :
un dispositif de mesure (4) comprenant au moins deux balances (10, 12) en communication avec un centre terminal (14), les balances (10, 12) comprenant un réceptacle pour contenir un produit ;
un centre de commande (16) en communication avec le centre terminal (14), le centre de commande (16) ayant la capacité de collecter et distribuer des données se rapportant à un produit retiré du réceptacle de chaque balance et un ordinateur (18) avec un logiciel pour organiser, analyser et afficher les données, dans lequel
chacune desdites au moins deux balances (10, 12) communique avec le centre de commande (16) uniquement par l'intermédiaire du centre terminal (14) et l'ordinateur (18) avec le logiciel pour organiser, analyser et afficher les données communique avec le centre terminal (14) uniquement par l'intermédiaire du centre de commande (16).

2. Système selon la revendication 1, dans lequel
a) chacun des réceptacles comprend un bol destiné à contenir un produit ; facultativement dans lequel le produit comprend un aliment pour animal de compagnie ; ou
b) chacune des balances (10, 12) comprend un composant choisi dans le groupe constitué d'un capteur de poids, un bloc d'alimentation par batterie, un affichage à cristaux liquides, une batterie rechargeable ou ordinaire, une carte à circuit, et une combinaison de ceux-ci ; ou
c) les balances (10, 12) sont fixées de façon amovible l'une à l'autre.

3. Procédé de collecte de données à distance, comprenant :
la fourniture d'un dispositif de mesure (4) comprenant au moins deux balances (10, 12) en communication avec un centre terminal (14), un centre de commande (16) et un ordinateur (18) avec un logiciel pour organiser, analyser et afficher les données,
dans lequel chacune desdites au moins deux balances (10, 12) communique avec le centre de commande (16) uniquement par l'intermédiaire du centre terminal (14) et l'ordinateur (18) avec le logiciel pour organiser, analyser et afficher les données communique avec le centre terminal (14) uniquement par l'intermédiaire du centre de commande (16), dans lequel chacune des balances (10, 12)comprend un produit ; et
la collecte de données par rapport à la quantité de produit retirée de chacune des balances (10, 12).

4. Procédé selon la revendication 3, dans lequel
a) le centre de commande (16) collecte, distribue et analyse les données ; ou
b) le procédé comprend l'affichage des données collectées sous la forme d'un graphique ; ou
c) les données sont collectées pendant une période de temps prédéterminée ; ou
d) le dispositif de mesure (4) se coupe automatiquement après une période de temps prédéterminée lorsqu'il n'est pas utilisé ; ou
e) le centre de commande (16) est capable d'effectuer une pluralité d'essais pour déterminer les quantités de produit retirées de chacune des balances ; ou
f) les données sont transférées du dispositif de mesure (4) au centre terminal (14) par le biais d'une connexion sans fil à basse fréquence ; ou
g) chacune des balances (10, 12) comprend un bol destiné à contenir le produit ; ou
h) chacune des balances (10, 12) comprend un composant choisi dans le groupe constitué d'un capteur de poids, un bloc d'alimentation par batterie, un affichage à cristaux liquides, une batterie rechargeable ou ordinaire, une carte à circuit, et leurs combinaisons ; ou
i) les balances (10, 12) sont fixées de façon amovible l'une à l'autre ; ou
j) le produit comprend un aliment pour animal de compagnie.

5. Procédé selon la revendication 3, dans lequel chacune des balances comprend en tant que produit un aliment différent pour animal de compagnie, le procédé comprenant en outre l'étape consistant à présenter à un animal de compagnie les aliments pour animal de compagnie sur les balances (10, 12),
dans lequel la collecte de données par rapport à la quantité de produit retirée de chacune des balances (10, 12) est
une collecte de données par rapport à la quantité d'aliment pour animal de compagnie mangée par l'animal de compagnie à partir de chacune des balances (10, 12).

6. Procédé selon la revendication 5, dans lequel le centre de commande (16) collecte et distribue les données.

7. Procédé selon la revendication 5, comprenant l'affichage des données collectées sous la forme d'un graphique.

8. Procédé selon la revendication 5, comprenant la collecte de données chaque seconde pendant une période de temps prédéterminée.

9. Procédé selon la revendication 5, comprenant la collecte de données chaque minute pendant une période de temps prédéterminée.
